# EUROPÄISCHE PATENTSCHRIFT

(11) **EP 0 769 534 B1**
(45) Veröffentlichungstag und Bekanntmachung des Hinweises auf die Patenterteilung: **12.09.2001**
(21) Anmeldenummer: 96116027.2
(22) Anmeldetag: 07.10.1996
(51) Int. Cl.: C09C 1/00, C09D 7/12, C08K 3/00, A61K 7/00, C04B 33/14, C03C 4/02

(54) **Verfahren zur Herstellung von Einschlusspigmenten**
Process for manufacturing occlusion pigments
Procédé de fabrication de pigments d'occlusion

(30) Priorität: 20.10.1995 DE 19539116
(43) Veröffentlichungstag der Anmeldung: 23.04.1997
(73) Patentinhaber: MERCK PATENT GmbH, 64293 Darmstadt (DE)
(72) Erfinder: Kuntz, Mathias, Dr., 64392 Ober-Beerbach (DE); Linne, Hubert, 64853 Otzberg (DE); Weitzel, Joachim, 64293 Darmstadt (DE); Heinz, Dieter, 64646 Heppenheim (DE); Riddle, Rodney, Dorset BH 12AG (GB)

(56) Entgegenhaltungen:
- EP-A- 0 371 211
- EP-A- 0 455 933
- EP-A- 0 507 165
- WO-A-90/14307

## Beschreibung

Die Erfindung betrifft ein Verfahren zur Herstellung von Einschlußpigmenten durch einen Sprühpyrolyseprozeß.

Verfahren zur Herstellung von Einschlußpigmenten sind bekannt. Sie wurden entwickelt, um witterungsstabile und temperaturstabile Pigmente herstellen zu können. Beispielsweise werden für Glasuren beim keramischen Dekorbrand oder für Porzellan Pigmente benötigt, die auch bis Temperaturen von 1500 °C stabil sind.

Perlglanzpigmente sind als Inglasurpigmente für den keramischen Dekorbrand nicht geeignet, da sie sich unter der Einwirkung der Netzwerkwandler Natrium und Kalium und von Flußhilfsmitteln wie Blei, Bor oder Wismut auch innerhalb kurzer Brennzyklen fast vollständig auflösen. Es ist deshalb notwendig, diese Pigmente durch eine zusätzliche Umhüllung für die oben genannte Anwendung einsetzbar zu machen.

Darüberhinaus ist es vorteilhaft, für manche Anwendungen mit Siliciumdioxid umhüllte Perlglanzpigmente zur Verfügung zu haben. Durch eine Hüllschicht aus SiO₂ wird beispielsweise die Stabilität gegen Vergilbung und die Stabilität in Wasserlacksystemen erhöht.

In DE 40 14 928 wird ein Verfahren zur Herstellung von umhüllten Spinell-Farbpigmente mit einem Kern aus einem farbigen Spinell und einer diesen Kern zumindest teilweise umhüllenden glasigen Schicht aus einem silikalischen Material beschrieben, indem man ein homogenes Pulvergemisch aus dem farbigen Spinell Siliciumdioxid und mindestens einem Mineralisator aus der Reihe der Alkali-, Erdalkali- und/oder Erdmetallhalogenide bei 900 bis 1300 °C 0,5 bis 5 Stunden sintert und das gesinterte Produkt naß oder trocken aufmahlt.

In EP 498 686 wird ein Verfahren zur Herstellung eines witterungsbeständigen Perlglanzpigmentes beschrieben, indem auf das Perlglanzpigment eine Schicht aus Cerhydroxid aufgefällt wird. Dazu wird das Perlglanzpigment (Wismutoxychlorid) in einer Cersalzlösung dispergiert und durch Änderung des pH-Wertes von 6,2 auf 10 Cerhydroxid auf dem plättchenförmigen Perlglanzpigment abgeschieden.

Diese Verfahren haben den Nachteil, daß beim Calcinieren des Pigmentes die Teilchen miteinander versintern und das Produkt erneut vermahlen werden muß. Dadurch wird aber die Umhüllung zum Teil wieder beschädigt und das angestrebte Ziel nur zum Teil erreicht. Außerdem hat sich gezeigt, daß mit diesem Verfahren ein vollständiger Einschluß der Pigmente mit dem oxidischen oder silikalischen Hüllmaterial, wie zum Beispiel ZrO₂, SnO₂, Al₂O₃ oder ZrSiO₄, praktisch nicht möglich ist.

Aufgabe der Erfindung ist es, ein Verfahren bereit zu stellen, mit dem eine vollständige Umhüllung der Pigmentteilchen erreicht und ein Agglomerieren der umhüllten Pigmentteilchen verhindert wird.

Aus EP 0 341 274 ist ein Verfahren zur Herstellung von Mehrelementmetalloxidpulvern zur Verwendung als Vorstufen für die Herstellung von hochtemperatur-supraleitenden Keramiken durch Vermischen von Metallsalzlösungen im für das gewünschte Endprodukt erforderlichen stöchiometrischen Verhältnis, Versprühen der homogenen Lösung in einen horizontal angeordneten röhrenartigen, dabei auf eine Temperatur von 800 bis 1100 °C erhitzten Ofen Fördern des Sprühnebels entlang der Hauptachse des Ofens mittels Heißluft und Auffangen des feinverteilten Metalloxidpulvers mittels eines Filters bekannt.

Aus DE 39 16 643 ist ein Verfahren zur Herstellung von oxidischen Keramikpulvern bekannt. Es handelt sich hierbei um ein pyrolytisches Verfahren zur Herstellung von oxidischen Keramikpulvern durch Verbrennung entsprechender Metallnitrate enthaltender wäßriger Lösungen in Gegenwart von als Brennstoff fungierenden organischen Substanzen oder elementarem Kohlenstoff, wobei die Menge an Nitrat in der Lösung auf die Menge an Brennstoff so abgestimmt wird, daß nach Zündung durch eine mindestens 250 °C heiße Zündquelle eine sich weitgehend selbst tragende Verbrennung abläuft, in der der Nitratsauerstoff zu mindestens 75 % zur vollständigen Verbrennung des Brennstoffes beiträgt. Nitratlösung und Brennstoff können als Gemisch oder getrennt in den Reaktionsraum gesprüht werden, wo durch die mindestens 250 °C heiße Zündquelle eine Zündung des Gemisches erfolgt.

Überraschenderweise wurde nun gefunden, daß bei Anwendung eines Sprühpyrolyseverfahrens Einschlußpigmente in der gewünschten Qualität hergestellt werden können, indem das zu verkapselnde Pigment in einer wäßrigen Lösung oder Suspension des Einschlu βmaterials suspendiert wird, die Suspension in einem horizontal angeordneten röhrenartigen Ofen bei 850 bis 1100 °C, vorzugsweise 850 bis 900 °C, versprüht wird, der Sprühnebel entlang der Hauptachse des Ofens mittels Heißluft gefördert wird und das Einschlußpigment mittels geeigneter Vorrichtungen, zum Beispiel mittels eines Filters, aufgefangen wird. Weitere Einzelheiten dieses Verfahrens sind in EP 0 341 274 näher beschrieben. Es ist auch unter der Bezeichnung EDS-Verfahren (Evaporative Decompositions of Solutions) bekannt. Nach einer weiteren Ausführungsform des erfindungsgemäßen Verfahrens werden die Einschlußpigmente in der gewünschten Qualität auch nach NPA-Verfahren hergestellt, indem das zu verkapselnde Pigment in einer wäßrigen Lösung oder Suspension eines Precursors des Einschlußmaterials suspendiert wird, der Pigmentsuspension Salpetersäure und gegebenenfalls Brennstoff zugesetzt wird, die Salpetersäure enthaltende Pigmentsuspension und der Brennstoff im Gemisch oder getrennt in einen Reaktionsraum gesprüht und dort durch eine mindestens 250 °C heiße Zündquelle gezündet werden und das erhaltene Einschlußpigment in geeigneten Vorrichtungen, wie zum Beispiel Filtern, Auffangkammern oder Zyklonen aufgefangen wird, wobei die Menge an Salpetersäure in der Pigmentsuspension auf die Menge an Brennstoff so abgestimmt wird, daß sich nach Zündung eine sich weitgehend selbst tragende Verbrennung abläuft, in der die Salpetersäure zu mindestens 75 % zur vollständigen Verbrennung des Brennstoffes beiträgt. Weitere Einzelheiten dieses Verfahrens sind in DE 39 16 643 beschrieben. Dieses Verfahren wird auch als NPA-Verfahren (Nitrat-Pyrolyse-Anlage) bezeichnet.

Weiterhin kann für die Herstellung der Einschlußpigmente das in EPO 0 681 989 beschriebene Verfahren unter Verwendung einer Wasserstoff/Sauerstoff-Flamme eingesetzt werden, indem die Suspension durch eine Wasserstoff/Sauerstoff-Flamme in einer solchen Weise gesprüht wird, daß eine Flammentemperatur von 800 bis 1100 °C aufrechterhalten wird, wobei ein Kontakt des Sprühnebels und des erzeugten Pigments während des Prozesses mit Kohlenstoff oder Kohlenstoff enthaltenden Verbindungen oder Materialien strikt vermieden wird.

Gegenstand der Erfindung ist außerdem die Verwendung der erfindungsgemäß hergestellten Pigmente zur Pigmentierung von Lacken, Druckfarben, Kunststoffen, Kosmetika und Glasuren für Keramiken und Gläser, wobei die Pigmente auch als Mischungen mit handelsüblichen Pigmenten eingesetzt werden.

Gegenstand der Erfindung sind auch Lacke, Druckfarben, Kunststoffe, Kosmetika und Glasuren für Keramiken und Gläser, die mit einem erfindungsgemäßen Pigment pigmentiert sind.

Als Einschlußmaterial (Hüllmaterial) für die Einschlußpigmente werden oxidische oder silikalische Materialien wie zum Beispiel ZrO₂, SnO₂, Al₂O₃, SiO₂ oder ZrSiO₄ verwendet, wobei ZrSiO₄ und SiO₂ bevorzugt sind. In der Ausgangssuspension werden Vorstufen (Precursor) der Hüllmaterialien eingesetzt. Darunter sind wasserlösliche Salze der oxydbildenden Metalle, wie zum Beispiel Zirkonylchlorid oder Zirkonylnitrat, oder Sole, wie zum Beispiel Zirkonoxid-Sol oder Siliciumdioxidsol, zu verstehen. Im Falle von ZrSiO₄ als Hüllmaterial müssen eine Zirkonkomponente (Zirkonylnitrat oder Zirkonoxidsol) und eine silikalische Komponente (Silikat oder Siliciumdioxidsol) als Precursor eingesetzt werden.

In der Ausgangssuspension können auch farbgebende Stoffe, wie Metallsalze oder Metalloxide suspendiert oder gelöst werden, wodurch auch farbige Überzüge erhalten werden können.

Die Suspension kann gegebenenfalls noch einen Mineralisator, beispielsweise Lithiumnitrat, enthalten. Der Mineralisator wird in einer Konzentration von 5 bis 50 Mol% bezogen auf das Hüllmaterial, beispielsweise ZrSiO₄, der Pigmentsuspension zugesetzt.

Die Dicke der Hüllschicht wird in Abhängigkeit vom Verwendungszweck des Einschlußpigmentes eingestellt. Für die Verwendung in Glasuren werden die Pigmente vorzugsweise mit einer Zirkonsilikatschicht eine Dicke von 10 bis 1000 nm umhüllt. Für die Verwendung der Pigmente in Wasserlacksystemen wird bevorzugt eine Siliciumdioxidschicht aufgebracht, die eine Dicke im Bereich von 5 bis 100 nm besitzt.

Als einzukapselnde Pigmente können anorganische und bedingt organische Pigmente verwendet werden. Es eignen sich sphärische Absorptionspigmente aber auch plättchenförmige Interferenzpigmente auf Basis Glimmer oder anderer plättchenförmiger Substrate, wie zum Beispiel Talk, Kaolin oder Glasplättchen und Metallpigmente.

Bevorzugte plättchenförmige Materialien sind Glimmer und plättchenförmige Pigmente, die gemäß der internationalen Anmeldung PCT/EP92/02 351 hergestellt werden. Sie bestehen aus einer transparenten, anorganischen plättchenförmigen Matrix, vorzugsweise Siliciumdioxid. Die Matrix wird durch Verfestigung eines flüssigen Precursors auf einem endlosen Band hergestellt. In diese Matrix können noch zusätzliche Inhaltsstoffe eingearbeitet werden.

Die plättchenförmigen Materialien haben typischerweise eine Dicke zwischen 0,05 und 5 µm und insbesondere zwischen 0,2 und 2 µm. Die Ausdehnung in den beiden anderen Dimensionen beträgt zwischen 1 und 250 µm und insbesondere zwischen 5 und 60 µm. Das Verhältnis der Ausdehnung in der Hauptdimension zur Dicke (aspect ratio) beträgt mehr als 3 und vorzugsweise mehr als 5.

Darüberhinaus ist es auch möglich, organische Pigmente, beispielsweise DPP-Rot oder Methylenblau, nach dem erfindungsgemäßen Verfahren einzukapseln um sie vor chemischen und mechanischen Angriffen zu schützen.

Die Pigmentkonzentration in der Ausgangssuspension liegt zwischen 1 und 50 Masse-%, bevorzugt zwischen 5 und 30 Masse-%. Sie ist abhängig von der gewünschten Dicke der Hüllschicht.

Als Brennstoffe für das erfindungsgemäße NPA-Verfahren kommen in erster Linie gasförmige Kohlenwasserstoffe, aliphatische oder aromatische Kohlenwasserstoffe oder aliphatische Alkohole, Kohlenstaub oder Gemische dieser Substanzen in Betracht. Die Anwendbarkeit ist jedoch nicht auf die in erster Linie aus wirtschaftlichen Gründen getroffene Auswahl beschränkt.

Gasförmige Kohlenwasserstoffe sind vorzugsweise Methan, Propan, Butan und Gemische hiervon. Erdgas, das im wesentlichen aus gasförmigen Kohlenwasserstoffen besteht, ist ein leicht verfügbarer und besonders wirtschaftlicher Brennstoff.

Unter aliphatischen oder aromatischen Kohlenwasserstoffen oder aliphatischen Alkoholen sind im Prinzip alle entsprechenden, gängigen, organischen Lösungsmittel zu verstehen, wie beispielsweise Pentan, Hexan, Benzol, Toluol, Xylol, Methanol, Ethanol, Isopropanol aber Kohlenwasserstoffgemische wie Leicht- und Schwerbenzine, Dieselöl etc. Als fester Brennstoff kommt vornehmlich Kohlenstaub in Betracht.

Wesentlich für das erfindungsgemäße Verfahren und alle seine Ausgestaltungsformen ist, daß die eingesetzten Brennstoffe alle durch eine mindestens 250 °C heiße Zündungsquelle entzündbar sind.

Die spezifische Verfahrensdurchführung kann unterschiedlich ausgestaltet werden und je nach Ausgangssituation den stofflichen Gegebenheiten optimal angepaßt werden.

Eine Variante besteht darin, die Nitratlösung mit dem Brennstoff zunächst innig zu vermischen und dann im Gemisch der Verbrennung zuzuführen. Diese Variante kommt insbesondere dann in Betracht, wenn flüssige Brennstoffe eingesetzt werden sollen. Kohlenstaub kann aber auch gegebenenfalls zusammen mit flüssigen Brennstoffen mit der Lösung dispergiert und so im Gemisch verbrannt werden. Optimal ist diese Methode der Gemischverbrennung, wenn wasserlösliche bzw. wassermischbare Brennstoffe, wie etwa aliphatische Alkohole, eingesetzt werden.

Eine andere Variante besteht darin, Nitratlösung und Brennstoff getrennt zuzuführen und erst bei der Verbrennung innig zu vermischen. Diese Variante ist dann zu bevorzugen, wenn es sich bei den Brennstoffen um nicht mit Wasser mischbare Flüssigkeiten wie Kohlenwasserstoffe handelt. Optimal ist diese Methode, wenn gasförmige Kohlenwasserstoffe als Brennstoff eingesetzt werden. Es ist dies eine besonders bevorzugte Ausführungsform des erfindungsgemäßen Verfahrens.

Eine weitere Variante besteht darin, sowohl Nitratlösung und Brennstoff im Gemisch als auch zusätzlichen Brennstoff getrennt der Verbrennung zuzuführen. Diese Variante kommt besonders dann in Betracht, wenn gleichzeitig gasförmige, flüssige und gegebenenfalls feste Brennstoffe eingesetzt werden sollen. Günstig läßt sich diese Methode handhaben, wenn vom eingesetzten Brennstoff etwa 10-20 Gew.% in fester, 50-70 Gew.% in flüssiger und 20-30 Gew.% in gasförmiger Form vorliegen.

Die erfindungsgemäßen Verfahren können in allen ihren Varianten grundsätzlich in Vorrichtungen bzw. Anlagen durchgeführt werden, wie sie vom Prinzip her von üblichen Sprühpyrolyseverfahren bekannt sind. Derartige Vorrichtungen und Anlagen bestehen meist aus einem Rohrreaktor, der im Prinzip aufgebaut ist aus einer Einlaßzone für das zu versprühende Medium, einer Reaktionszone, in der der Pyrolysevorgang abläuft und einer Auslaßzone, die in eine Vorrichtung zum Abscheiden des Reaktionsproduktes mündet. Die Einlaßzone besteht hierbei meist aus einer oder mehrerer Düsen, durch die das Medium, gegebenenfalls auch gesteuert, versprüht wird. Die Reaktionszone ist meist indirekt durch einen Ofen oder direkt durch zugeführte heiße Verbrennungsgase beheizt. Das Auffangen des Reaktionsproduktes erfolgt meist durch Filter, Auffangkammern, einen oder mehrere Zyklone. Dem einschlägigen Fachmann sind die entsprechenden Anlagen und technischen Möglichkeiten geläufig und er kann sie problemlos auf die spezifischen Gegebenheiten des erfindungsgemäßen Verfahrens anwenden. Die Dimensionierung einer entsprechenden Anlage ist abhängig von der gewünschten Produktionskapazität und der Fahrweise, also ob teil- oder dauerkontinuierlicher Betrieb vorgesehen ist.

Für die Durchführung des NPA-Verfahrens ist es erforderlich, daß das in den Reaktor gesprühte bzw. sich dort bildende Lösungs-Brennstoff-Gemisch gezündet wird, so daß sich die Reaktion in Form einer sich selbst tragenden, vollständigen Verbrennung einstellt. Hierzu muß das Gemisch in Kontakt mit einer mindestens 250 °C heißen Zündquelle kommen. Als derartige Zündquellen kommen beispielsweise in Betracht die entsprechend von außen aufgeheizte Rohrwandung des Reaktors oder separat im Reaktor installierte Zündquellen wie Glühkerzen, Zündkerzen, Zündflamme, elektrisch beheizte Glühdrähte oder Glühgitter. Als besonders zweckmäßig und wirksam erweist sich die Zündung mittels eines mit Gas und Luft betriebenen Zündbrenners.

Wenn die Reaktion angefahren ist, d.h. nach Zündung des Reaktionsgemisches und Ausbildung einer konstanten Verbrennungsreaktion bei kontinuierlicher Zufuhr des Reaktionsgemisches, stellen sich im Reaktor schnell derartig hohe Temperaturen ein, typisch über 1000 °C, daß ein weiterer Betrieb der Zündungsquelle normalerweise entbehrlich ist. Lediglich zur Betriebssicherheit, etwa in kritischeren Einzelfällen, kann es aber sinnvoll sein, die Zündungsquelle, vorzugsweise in Form einer Stützflamme, weiter zu betreiben.

Für die besonders bevorzugte Variante des erfindungsgemäßen NPA-Verfahrens, nämlich der Verbrennung der Nitratlösung in Gegenwart von gasförmigen Kohlenwasserstoffen als Brennstoff, ist es zweckmäßig, die Gaszuführung gleich in Form eines Gasbrenners zu gestalten, in dessen Flammenzone die Nitratlösung eingesprüht wird. Für das Anfahren des Betriebes sorgt man durch entsprechende Luftzufuhr zunächst für eine Zündung und Verbrennung des Brenngases und regelt nach Zuschaltung der Lösungseinsprühung die Gasmengen-, Luftmengen- und Lösungsmengenzufuhr so ein, daß durch die Pyrolyse der Nitratlösung im wesentlichen die Verbrennung des Brenngases erfolgt. Diese Regelung läßt sich beispielsweise günstig mit Hilfe einer im Reaktor installierten Lambda-Sonde erreichen, die im Reaktionsabgas den Sauerstoffpartialdruck mißt, anhand dessen sich eine im wesentlichen stöchiometrische Verbrennung einstellen läßt.

Besonders geeignet für die Durchführung des NPA-Verfahrens ist ein Reaktor, der aus einem horizontal angeordneten Rohr von 300 cm Länge und 20 cm Innendurchmesser besteht. Am Kopfende befindet sich ein Gasbrennerkopf, dessen Düsen mit Propan-Butan-Gemisch und Luft gespeist werden, mit elektrischer Zündung. Durch eine im Brennerkopf zentral angeordnete Düse wird die Pigmentsuspension in den Flammenkegel eingesprüht.

Im rückwärtigen Bereich des Reaktors ist eine Lambdasonde installiert, mit deren Hilfe über die Regelung der Gas-/Luft-Zufuhr die Reaktoratmosphäre eingestellt werden kann.

Das Einschlußpigment wird in nachgeschalteten Kammerfiltern gesammelt.

Ein Reaktor, der für die Durchführung des erfindungsgemäßen Verfahrens geeignet ist, ist ein Sprühröstreaktor, wie er in der EP 0 341 274 näher beschrieben wird. Es handelt sich hierbei um einen horizontal angeordneten Rohreaktor, der auf 850 bis 1100 °C aufgeheizt wird. An einem Ende des Rohres befindet sich ein Sprühknopf, dem die Pigmentsuspension und Heißluft unter einem Druck von 0,34-0,52 bar zugeführt werden. Am anderen Ende des Rohres befindet sich eine Auffangvorrichtung, beispielsweise ein Filter, um das gebildete Einschlußpigment aus dem Gasstrom zu entfernen.

Gegebenenfalls ist eine Nachcalcinierung des erhaltenen Einschlußpigmentes erforderlich. Bei Verwendung von Zirkonsilikat als Einschlußmaterial ist eine Temperatur von 816 °C für eine vollständige Phasenumwandlung erforderlich. Wenn die Pigmentpartikel im Reaktor nicht auf diese Temperatur aufgeheizt werden, ist eine anschließende Calcinierung bei mindestens 816 °C erforderlich, um eine vollständige Phasenumwandlung in Zirkonsilikat zu erreichen.

Das erfindungsgemäße Verfahren hat den großen Vorteil, daß die erhaltenen Einschlußpigmente nicht mehr gemahlen werden müssen. Die gewünschte Korngröße und die Korngrößenverteilung kann durch eine entsprechend gewählte Fraktion des eingesetzten Pigmentes eingestellt werden. Die Pigmente sind vollständig mit der Hüllschicht überzogen. Das trifft überraschenderweise auch auf plättchenförmige Pigmente zu, was vom Fachmann nicht erwartet werden konnte.

Die nachfolgenden Beispiele sollen die Erfindung erläutern, ohne sie zu begrenzen.

### Beispiel 1

500 g Iriodin® 306 werden in einem Gemisch aus 471 g Natriumwasserglas und 4,6 I Wasser suspendiert. Die erhaltene Suspension, die ein Volumen von 5 l besitzt, wird während des Versprühens in einem Sprühröstreaktor ständig gerührt. Dazu wird sie mit einer Dosierrate von 1 l/h durch eine Zweistoffdüse gepumpt und dabei mit Druckluft von 2 bar zerstäubt. Das erhaltene Aerosol wird durch einen auf 850-900 °C erhitzten Rohrreaktor geführt. Nach Passieren der Reaktionszone wird der Produktstrom in eine evakuierte Kammer geleitet und das Produkt auf einem Filter aus Sinterstahl abgeschieden. Im Verlauf von 3 Stunden werden 25 g Produkt aus 2 l Pigmentsuspension erhalten.

Das Einschlußpigment besitzt eine 100 nm dicke Schicht aus Siliciumdioxid. Die Dicke der Schicht wurde optisch im Rasterelektronenmikroskop bestimmt, indem umhüllte Pigmentteilchen in Lack eingebettet und gebrochen wurden.

### Beispiel 2

100 g Iriodin® 306 werden in einem Gemisch aus 27,5 g Ludox und 2 I Wasser suspendiert. Die erhaltene Suspension, die ein Volumen von 2,5 I besitzt, wird ständig gerührt, um ein Absetzen des Pigmentes zu verhindern. Die Suspension wird mit einer Dosierrate von 1 l/h durch eine Zweistoffdüse gepumpt und dabei mit Druckluft von 1 bar zerstäubt. Das erhaltene Aerosol wird durch einen auf 850-900 °C erhitzten Rohreaktor geführt. Nach Passieren der Reaktionszone wird der Produktstrom in eine evakuierte Kammer geleitet und das Einschlußpigment auf einem Filter aus Sinterstahl abgeschieden. Im Verlauf von 4 Stunden werden 34 g Produkt erhalten.

Die Dicke der Siliciumdioxidschicht beträgt 25 nm.

### Beispiel 3

100 g Iriodin® 306 werden in einem Gemisch aus 10,3 g Ludox, 15,7 g ZrO(NO₃)₂, 28,5 g LiNO₃ · 3 H₂O und 2 I Wasser suspendiert. Die erhaltene Suspension, die ein Volumen von 2,5 I besitzt, wird ständig gerührt, um ein Absetzen des Pigmentes zu verhindern. Die Suspension wird mit einer Dosierrate von 1 l/h durch eine Zweistoffdüse gepumpt und dabei mit Druckluft von 1 bar zerstäubt. Das erhaltene Aerosol wird durch einen auf 850-900 °C erhitzten Rohreaktor geführt. Nach Passieren der Reaktionszone wird der Produktstrom in eine evakuierte Kammer geleitet und das Einschlußpigment auf einem Filter aus Sinterstahl abgeschieden. Im Verlauf von 5 Stunden werden 12 g Produkt erhalten, das anschließend in einem Röhrenofen bei 816 °C für 30 Minuten calciniert wird. Eine XRD-Analyse des calcinierten Produktes zeigt die Anwesenheit einer Zirkonsilikatphase. Die Dicke der Zirkonsilikatschicht beträgt 25 nm.

### Beispiel 4

50 g Iriodin® 306 werden in einem Gemisch aus 206,3 g Ludox, 380,2 g ZTO(NO₃)₂, 109,5 g LiNO₃ · 3 H₂O und 2 l Wasser suspendiert. Die erhaltene Suspension, die ein Volumen von 2,5 l besitzt, wird ständig gerührt, um ein Absetzen des Pigmentes zu verhindern. Die Suspension wird mit einer Dosierrate von 1 l/h durch eine Zweistoffdüse gepumpt und dabei mit Druckluft von 1 bar zerstäubt. Das erhaltene Aerosol wird durch einen auf 850-900 °C erhitzten Rohreaktor geführt. Nach Passieren der Reaktionszone wird der Produktstrom in eine evakuierte Kammer geleitet und das Einschfußpigment auf einem Filter aus Sinterstahl abgeschieden. Im Verlauf von 6 Stunden werden 126 g Produkt erhalten, das anschließend in einem Röhrenofen bei 816 °C für 30 Minuten calciniert wird.

Eine XRD-Analyse zeigt die Anwesenheit einer Zirkonsilikatphase. Die Dicke der Zirkonsilikatschicht beträgt 400 nm.

### Beispiel 5

10 kg Iriodin® 306 werden in einem Gemisch aus 41,2 kg Ludox, 76,04 kg Zirkonylnitratlösung, 21,9 kg Lithiumnitrat und 400 kg 65 %iger Salpetersäure suspendiert und die erhaltene Suspension ständig gerührt. Die Suspension wird mit einer Dosierrate von 15-30 kg/h in den Flammenkegel eines NPA-Reaktors gesprüht. Vor dem Einsprühen in den Reaktor wurde über einen Statikmischer Essigsäure zudosiert. Insgesamt werden 202 kg Essigsäure benötigt. Dabei wurde ein leichter Unterschuß an Reduktionsmittel (Essigsäure) im Vergleich zur eingesetzten Nitratmenge eingestellt. Der zur stöchiometrischen Verbrennung erforderliche Restreduktionsmittelanteil wurde über die Flammeneinstellung erreicht und über eine λ-Sonde am Ende des Rohreaktors überprüft. Der Prozeß wurde über ein Thermoclement überwacht und gesteuert und die Temperatur im Reaktor bei 1000 bis 1300 °C gehalten.

Das erhaltene Produkt wurde gecoatet und wies eine Zirkonsilikatphase auf.

## Patentansprüche

1. Pyrolytisches Verfahren zur Herstellung von Einschlußpigmenten durch Suspendieren des zu verkapselnden Pigmentes in einer wäßrigen Lösung oder Suspension einer Vorstufe des Einschlußmaterials, Versprühen der homogenen Lösung in einen horizontal angeordneten, röhrenartigen, auf 800 bis 1100 °C erhitzten Ofen, Fördern des Sprühnebels entlang der Hauptachse des Ofens mittels Heißluft und Abscheiden des erhaltenen Einschlußpigmentes aus dem Produktstrom mittels einer Abscheidevorrichtung.

2. Pyrolytisches Verfahren zur Herstellung von Einschlußpigmenten nach Anspruch 1 durch Suspendieren des zu verkapselnden Pigmentes in einer wäßrigen Lösung oder Suspension einer Vorstufe des Einschlußmaterials, Zusetzen von Salpetersäure und Brennstoff zur erhaltenen Suspension, Versprühen der Suspension und des Brennstoffes im Gemisch oder getrennt in einem Reaktionsraum, Zünden des Gemisches durch eine mindestens 250 °C heiße Zündquelle und Abscheiden des erhaltenen Einschlußpigmentes aus dem Produktstrom durch geeignete Trennvorrichtungen, wobei die Menge an Salpetersäure in der Pigmentsuspension auf die Menge an Brennstoff so abgestimmt wird, daß sich nach Zündung eines sich weitgehend selbst tragende Verbrennung abläuft, in der die Salpetersäure zu mindestens 75 % zur vollständigen Verbrennung des Brennstoffes beiträgt.

3. Pyrolytisches Verfahren zur Herstellung von Einschlußpigmenten durch Suspendieren des zu verkapselnden Pigmentes in einer wäßrigen Lösung oder Suspension einer Vorstufe des Einschlußmaterials, Sprühen der Suspension durch eine Wasserstoff/Sauerstoff-Flamme in einer solchen Weise, daß eine Flammentemperatur von 800 bis 1100 °C aufrechterhalten wird, wobei ein Kontakt des Sprühnebels und des erzeugten Pigmentes während des Prozesses mit Kohlenstoff oder Kohlenstoff enthaltenden Verbindungen oder Materialien strikt vermieden wird.

4. Verfahren nach den Ansprüchen 1 und 3, **dadurch gekennzeichnet**, daß das erhaltene Einschlußpigment anschließend bei mindestens 816 °C für 10 bis 50 Minuten geglüht wird.

5. Verfahren nach mindestens einem der Ansprüche 1 bis 4, **dadurch gekennzeichnet, daß** als Vorstufen des Einschlußmaterials wasserlösliche Salze und Oxidhydratsole der Elemente Zirkon, Silicium, Zinn und Aluminium eingesetzt werden.

6. Verfahren nach mindestens einem der Ansprüche 1 und 2, **dadurch gekennzeichnet, daß** als Brennstoff gasförmige Kohlenwasserstoffe, aliphatische oder aromatische Kohlenwasserstoffe oder aliphatische Carbonsäuren, aliphatische Alkohole, Kohlenstaub oder Gemische hiervon eingesetzt werden.

7. Verwendung der Pigmente nach den Ansprüchen 1 bis 6 zur Pigmentierung von Lacken, Druckfarben, Kunststoffen, Kosmetika, Glasuren für Keramiken und Gläser.

8. Verwendung nach Anspruch 7, **dadurch gekennzeichnet, daß** die Pigmente als Mischungen mit handelsüblichen Pigmenten eingesetzt werden.

9. Lacke, Druckfarben, Kunststoffe, Kosmetika, Keramiken und Gläser, welche mit einem Pigment nach den Ansprüchen 1 bis 6 pigmentiert sind.

## Claims

1. Pyrolytic process for the preparation of inclusion pigments by suspending the pigment to be encapsulated in an aqueous solution or suspension of a precursor of the inclusion material, spraying the homogeneous solution into a horizontal tubular furnace heated at from 800 to 1100°C, conveying the spray mist along the principal axis of the furnace by means of hot air and separating the resulting inclusion pigment from the product stream by means of a separating device.

2. Pyrolytic process for the preparation of inclusion pigments according to Claim 1, by suspending the pigment to be encapsulated in an aqueous solution or suspension of a precursor of the inclusion material, adding nitric acid and fuel to the suspension obtained, spraying the suspension and fuel, in a mixture or separately, in a reaction chamber, igniting the mixture by means of an ignition source which has a temperature of at least 250°C and separating the resulting inclusion pigment from the product stream by means of suitable separation devices, the quantity of nitric acid in the pigment suspension being matched to the quantity of fuel such that combustion is largely self-supporting after ignition, at least 75% of the nitric acid contributing to complete combustion of the fuel.

3. Pyrolytic process for the preparation of inclusion pigments by suspending the pigment to be encapsulated in an aqueous solution or suspension of a precursor of the inclusion material, spraying the suspension through a hydrogen/oxygen flame such that a flame temperature of from 800 to 1100°C is maintained, during which process contact of the spray mist and the pigment produced with carbon or carbon compounds or materials is rigorously avoided.

4. Process according to Claims 1 and 3, **characterized in that** the inclusion pigment obtained is subsequently calcined at at least 816°C for from 10 to 50 minutes.

5. Process according to at least one of Claims 1 to 4, **characterized in that** precursors of the inclusion material which are employed are water-soluble salts and oxide hydrate sols of the elements zirconium, silicon, tin and aluminium.

6. Process according to at least one of Claims 1 and 2, **characterized in that** fuels employed are gaseous hydrocarbons, aliphatic or aromatic hydrocarbons or aliphatic carboxylic acids, aliphatic alcohols, coal dust or mixtures thereof.

7. Use of the pigments according to Claims 1 to 6 for pigmenting paints, printing-inks, plastics, cosmetics, glazes for ceramics and glasses.

8. Use according to Claim 7, charaterized in that the pigments are employed as mixtures with commercially customary pigments.

9. Paints, printing-inks, plastics, cosmetics, ceramics and glasses, pigmented with a pigment according to Claims 1 to 6.

## Revendications

1. Procédé pyrolytique pour la fabrication de pigments d'inclusion par mise en suspension du pigment à encapsuler dans une solution ou suspension aqueuse d'un précurseur du matériau d'inclusion, par vaporisation de la solution homogène dans un four, de forme tubulaire, disposé horizontalement, chauffé à une température comprise entre 800 et 1.100°C, par transport du nébulisat le long de l'axe principal du four à l'aide d'air chaud et par séparation du pigment d'inclusion obtenu du courant réactionnel à l'aide d'un dispositif séparateur.

2. Procédé pyrolytique pour la fabrication de pigments d'inclusion selon la revendication 1 par mise en suspension du pigment à encapsuler dans une solution ou suspension aqueuse d'un précurseur du matériau d'inclusion, par addition d'acide nitrique et d'un combustible à la suspension obtenue, par vaporisation de la suspension et du combustible en mélange ou séparé dans un milieu réactionnel, par inflammation du mélange par une source d'inflammation à au moins 250°C et par séparation du pigment d'inclusion obtenu du courant réactionnel par des dispositifs séparateurs appropriés, la quantité d'acide nitrique dans la suspension de pigments étant réglée par rapport à la quantité de combustible de manière qu'après l'inflammation se déroule une combustion s'auto-entretenant largement, dans laquelle l'acide nitrique contribue pour au moins 75 % à la combustion complète du combustible.

3. Procédé pyrolytique pour la fabrication de pigments d'inclusion par mise en suspension du pigment à encapsuler dans une solution ou suspension aqueuse d'un précurseur du matériau d'inclusion, par vaporisation de la suspension par une flamme hydrogène/oxygène de façon telle que la température de la flamme soit maintenue entre 800 et 1.100°C, un contact du nébulisat et du pigment produit pendant l'opération avec le carbone ou des composés ou matériaux contenant du carbone étant rigoureusement évité.

4. Procédé selon les revendications 1 et 3, **caractérisé en ce que** le pigment d'inclusion obtenu est ensuite calciné à au moins 816°C pendant 10 à 50 minutes.

5. Procédé selon au moins l'une des revendications 1 à 4, **caractérisé en ce que** l'on utilise, comme précurseur du matériau d'inclusion, des sels solubles dans l'eau et des sols d'oxyhydrates des éléments zirconium, silicium, étain et aluminium.

6. Procédé selon au moins l'une des revendications 1 et 2, **caractérisé en ce que** l'on utilise, comme combustible, des hydrocarbures gazeux, des hydrocarbures aliphatiques ou aromatiques ou des acides carboxyliques aliphatiques, des alcools aliphatiques, de la poussière de charbon ou des mélanges de ceux-ci.

7. Utilisation des pigments selon les revendications 1 à 6 pour la pigmentation des vernis, des encres d'imprimerie, des matières plastiques, des cosmétiques, des glaçures pour céramiques et vernis.

8. Utilisation selon la revendication 7, **caractérisée en ce que** l'on utilise les pigments sous forme de mélanges avec des pigments courants dans le commerce.

9. Vernis, encres d'imprimerie, matières plastiques, cosmétiques, céramiques et verres, pigmentés avec un pigment selon les revendications 1 à 6.
